# EUROPÄISCHE PATENTSCHRIFT

(11) **EP 1 195 708 B1**
(45) Veröffentlichungstag und Bekanntmachung des Hinweises auf die Patenterteilung: **26.04.2006**
(21) Anmeldenummer: 01123627.0
(22) Anmeldetag: 02.10.2001
(51) Int. Cl.: G06F 19/00, A61M 1/16, A61M 1/34, A61M 1/36

(54) **Extracorporales Blutbehandlungssystem**
Extracorporeal blood treatment system
Systeme de traitement extracorporel du sang

(30) Priorität: 05.10.2000 DE 10049393
(43) Veröffentlichungstag der Anmeldung: 10.04.2002
(73) Patentinhaber: B. Braun Medizintechnologie GmbH, 34212 Melsungen (DE)
(72) Erfinder: Müller, Friedrich, 35792 Löhnberg (DE); Dolgos, Sándor Dr., 2000 Szentendre (HU); Szamkó, Péter, 2131 Göd (HU)
(74) Vertreter: Selting, Günther

(56) Entgegenhaltungen:
- EP-A- 0 890 919
- WO-A-01/37899
- WO-A-01/65463
- US-A- 5 891 035
- US-A- 6 035 328

## Beschreibung

Die Erfindung betrifft ein extracorporales Blutbehandlungssystem mit einer ECB-Station (ExtraCorporales Blut), die eine ECB-Einrichtung enthält.

Zur extracorporalen Blutbehandlung gehören u.a. Hämodialyse, Hämofiltration und Hämodiafiltration. Solche Blutbehandlungen werden in speziellen Maschinen durchgeführt, an die der Patient vorübergehend angeschlossen wird. Dabei wir dem Patienten Blut entnommen, das in einem extracorporalen Kreislauf über eine Behandlungseinrichtung geführt wird und anschließend dem Patienten wieder zugeführt wird. Solche ECB-Einrichtungen sind aufwendige Maschinen mit sehr komplexen Regelungen und Steuerungen. In Abhängigkeit von der durchzuführenden Blutbehandlung und in Abhängigkeit von den patientenbezogenen Parametern und medikamentenbezogenen Parametern müssen unterschiedliche Durchflussraten, Ultrafiltrationsprofile, Flüssigkeitskonzentrationen und andere Parameter eingestellt und überwacht werden. Hinzu kommt die Überwachung der Maschine auf interne oder externe Fehler. Die Bedienung von ECB-Einrichtungen erfordert eine umfassende Ausbildung. Entsprechendes gilt auch für die Wartung und Kontrolle von ECB-Einrichtungen, wozu hochqualifiziertes Personal erforderlich ist.

ECB-Einrichtungen haben ein Benutzer-Interface, das beispielsweise als Touchscreen ausgebildet sein kann, wodurch die Bedienung der Maschine erleichtert wird. Eine solche ECB-Einrichtung ist in dem US-Patent 5,788,851 beschrieben. Der Oberbegriff des Patentanspruchs 1 entspricht im Wesentlichen dieser Druckschrift.

Die US-Patente 5,715,823 und 5,891,035 beschreiben jeweils ein Ultraschall-Diagnose-System, bei dem eine Ultraschalleinheit an ein Datennetz, z.B. das Internet, angeschlossen ist. Von allen Stellen des Datennetzes aus besteht Zugriff auf die Ultraschalleinheit. Auf diese Weise können Mediziner eine Ultraschalluntersuchung verfolgen und bewerten, auch wenn sie weit entfernt vom Patientenort sind. Die Station, an der die Ultraschalleinheit sich befindet, enthält einen Browser und einen Web-Server sowie die entsprechende Ethernet-Hardware für den Anschluss an das Datennetz.

In US-A-6,035,328 ist ein medizin-therapeutisches und/oder diagnostisches System beschrieben, das in einem Behandlungsraum eine Betriebseinheit und in einem Kontrollraum eine weitere Betriebseinheit aufweist. Beide Betriebseinheiten korrespondieren über ein Leitungssystem, das durch einen Technikraum führt. Von diesem Leitungssystem zweigt ein HTTP-Server ab, der mit einem Intranet und über dieses auch mit dem Internet verbindbar ist. Die Betriebseinheiten korrespondieren untereinander nicht über einen Browser.

EP 0 890 919 A1 beschreibt ein medizinisches Messwerterfassungssystem, bei dem eine Gesundheits-Datenbank einen Webserver enthält, um über diesen über ein Kommunikationsnetzwerk mit einem externen Webbrowser zu kommunizieren. Auch hier dient der Browser ausschließlich zur externen Kommunikation.

Der Erfindung liegt die Aufgabe zugrunde, ein extracorporales Blutbehandlungssystem zu schaffen, bei dem auf eine ECB-Einrichtung sowohl von einem Benutzer-Interface als auch von einer entfernten Stelle aus zugegriffen werden kann, wobei die Datenübertragung vereinheitlicht und die Zugriffskontrolle erleichtert ist.

Die Lösung dieser Aufgabe erfolgt erfindungsgemäß mit den im Patentanspruch 1 angegebenen Merkmalen. Hiernach enthält die ECB-Station außer der ECB-Einrichtung (ECB = extracorporale Blutbehandlung) einen internen Web-Server und einen internen Browser. Der Web-Server ist eine Software, die Informationen über die ECB-Einrichtung derart bereitstellt, dass die Information über das Datennetz übertragen werden kann. Der Browser ist eine Software, die die Datenübertragung des Web-Servers kontrolliert. Die ECB-Station ist in der Weise konfiguriert, dass der interne Web-Server sowohl mit dem internen Browser als auch mit externen Browsern des Datennetzes oder dem Browser einer anderen ECB-Station kommunizieren kann. Alternativ oder zusätzlich kann der interne Browser sowohl mit dem internen Web-Server als auch mit externen Web-Servern oder dem Web-Server einer anderen ECB-Station kommunizieren. Die Besonderheit besteht darin, dass der Datenverkehr zwischen dem Benutzer-Interfacce und der ECB-Einrichtung über den internen Browser abgewickelt wird. Dies bedeutet, dass die Signale des Benutzer-Interface auf den Standard des Datennetzes konvertiert werden. Dem internen Web-Server werden die Signale des Benutzer-Interface in derselben Weise und in demselben Datenformat zugeführt wie die aus dem Datennetz kommenden Daten dem internen Web-Server zugeführt werden. Die Folge davon ist, dass die ECB-Einheit sämtliche Signale für Abfragen, Einstellungen, Befehle und andere Funktionen vom Web-Server empfängt, unabhängig davon, ob diese Daten von einer internen oder externen Quelle, beispielsweise einer externen ECB-Station geliefert wurden.

Die Erfindung ist sowohl in lokalen Datennetzen als auch in globalen Datennetzen anwendbar. Sie ermöglicht es, beispielsweise mehrere Dialysemaschinen von einer Zentrale aus zu überwachen unter Benutzung des lokalen Datennetzes des Krankenhauses, in dem sich mehrere Dialysemaschinen und die Zentrale befinden. Natürlich können auch mehrere Krankenhäuser untereinander vernetzt sein. Die Erfindung ermöglicht andererseits auch eine Kommunikation über ein universelles Datennetz, wobei beispielsweise die ECB-Einrichtung Zugriff auf eine Medikamenten-Datenbank hat, in der die Daten einer Vielzahl von Medikamenten enthalten sind. Beispielsweise kann einem bestimmten Medikament ein Infusionsprofil mit einer zeitlich variierenden Infusionsrate zugeordnet sein, wobei die Infusionsrate in Abhängigkeit von dem Körpergewicht des Patienten oder anderen Parametern eingestellt werden muss. Alle diese medikamentenbezogenen Daten können in einer zentralen Datenbank enthalten sein, auf die die ECB-Stationen zugreifen können.

Darüber hinaus besteht die Möglichkeit, dass ein Service-Center eingerichtet wird, an dem ein ausgebildeter Service-Techniker den Lauf einer ECB-Einrichtung verfolgt und Einstellungen an der Maschine vornehmen kann. Natürlich darf der Techniker nicht in eine laufende ECB-Behandlung eingreifen. Daher werden verschiedene Autorisationen gesetzt, d.h. im Web-Server wird festgelegt, welche Autorisierung ein Befehl, hier nach Herkunft des Befehls, hat. So ist es beispielsweise möglich, dass das Klinikpersonal am Benutzer-Interface die Infusionsrate verändert, dass der Service-Techniker am entfernten Ort hierzu jedoch keine Autorisierung hat. Der Service-Techniker kann beispielsweise in einem Service-Center arbeiten, das über einen Browser verfügt, oder an einer anderen ECB-Station oder auch an der zu überwachenden ECB-Station.

Bei dem erfindungsgemäßen Blutbehandlungssystem werden die Signale des Benutzer-Interface gewissermaßen auf den Standard des Datennetzes angehoben und über den internen Browser an den internen Web-Server geliefert. Dadurch werden sämtliche Signale, unabhängig von ihrer Herkunft, ihrem Ursprung, ihrem Zielort oder ihrem Bestimmungsort, in gleicher Weise verarbeitet, jedoch können je nach Bedarf unterschiedliche Autorisierungen für diese Signale erteilt werden.

Die Erfindung ermöglicht es auch, über das Benutzer-Interface und den Browser andere Daten über das Datennetz zu übertragen, beispielsweise Röntgenbilder oder patientenbezogene Informationen, um eine ärztliche Kapazität, die sich am entfernten Ort befindet und die Blutbehandlung verfolgt, umfassend über den Patienten zu informieren. So kann beispielsweise über den internen Browser ein E-Mail verschickt werden. Es besteht auch die Möglichkeit, Software-Versionen zu verschicken oder von einer entfernten Quelle herunterzuladen.

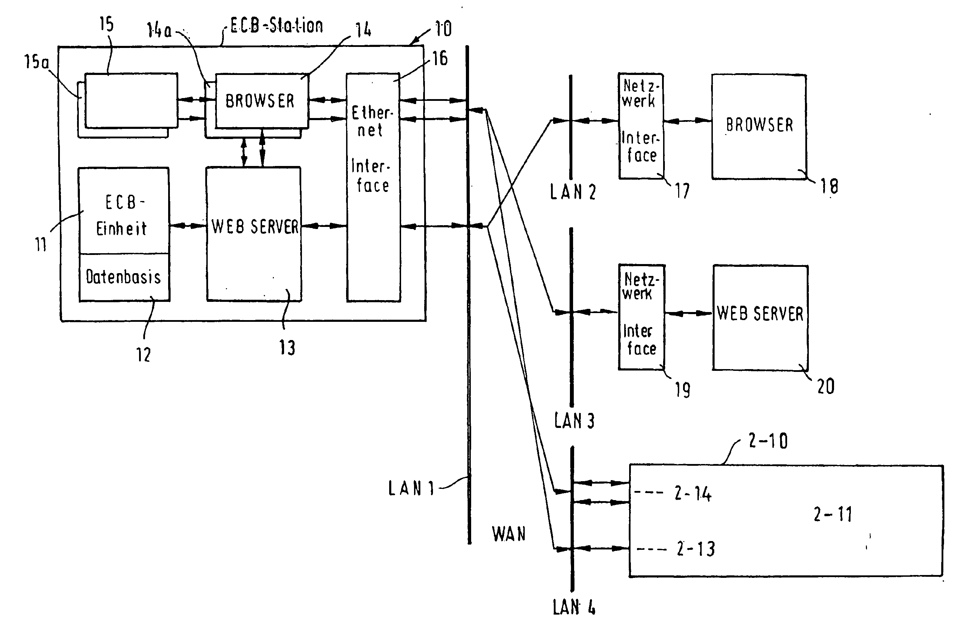

Im folgenden wird unter Bezugnahme auf die einzige Figur der Zeichnung ein Ausführungsbeispiel der Erfindung näher erläutert. In der Zeichnung ist ein Blockschaltbild eines extracorporalen Blutbehandlungssystems dargestellt.

Das Blutbehandlungssystem weist eine ECB-Station 10 auf, die eine ECB-Einheit 11 enthält. Die ECB-Einheit 11 ist beispielsweise eine Dialysemaschine einschließlich der zugehörigen Steuerungen, beispielsweise das Gerät "Dialog" der Firma B.Braun Melsungen AG. Der ECB-Einheit 11 ist eine Datenbasis 12 zugeordnet. Hierbei handelt es sich um einen Datenspeicher, der Behandlungskurven, Behandlungsparameter und maschinenbezogene Daten enthält. Die Steuereinheit der ECB-Einrichtung hat Zugriff auf die Datenbasis 12.

Die ECB-Einheit 11 kommuniziert mit dem internen Web-Server 13. Der Web-Server 13 ist ein Informationslieferant, der Informationen liefert, die für die Übertragung über das Datennetz geeignet sind. Der Web-Server 13 kommuniziert mit dem internen Browser 14. Der Browser 14 ist eine Software zur Steuerung der Datenübertragung über das Datennetz. Der Browser 14 kommuniziert seinerseits mit einem Benutzer-Interface 15. Hierbei handelt es sich um eine Ein-Ausgabevorrichtung, über die der Benutzer, d.h. das Klinikpersonal, Daten und Befehle eingeben kann, wobei als Ausgabevorrichtung beispielweise ein Bildschirm vorgesehen. Das Benutzer-Interface 15 ist beispielsweise ein Touchscreen.

Der interne Web-Server 13 und der interne Browser 14 kommunizieren über ein Netzwerk-Interface, z.B. ein Ethernet-Interface 16, mit einem Datennetz, bei dem es sich hier um ein lokales Datennetz LAN 1 (Local Area Network) handelt. Das Datennetz LAN 1 ist beispielsweise das Datennetz innerhalb einer Abteilung eines Hospitals. Das lokale Datennetz ist seinerseits Bestandteil eines globalen oder universalen Datennetzes WAN (Wide Area Network). Im Ausführungsbeispiel sind weitere lokale Netze LAN 2, LAN 3, LAN 4 vorgesehen, die ebenfalls Bestandteil des Datennetzes WAN sind.

An eines der lokalen Datennetze LAN 2 ist über ein Netzwerk-Interface 17 ein externer Browser 18 angeschlossen. Der Begriff "extern" bedeutet, dass der Browser nicht in der ECB-Einheit 10 angeordnet ist, die in der Zeichnung links dargestellt ist. Der externe Browser 18 kommuniziert über die geschilderten Datennetze mit dem internen Web-Server 13, um aus diesem Informationen oder Befehle abzurufen oder solche in ihn einzugeben. Der externe Browser 18 befindet sich beispielsweise in einem Service-Zentrum des Herstellers der ECB-Einheit 11. Der Service-Techniker kann die aktuelle Behandlung oder frühere Behandlungen, die mit der ECB-Einheit durchgeführt werden, verfolgen und technische Unterstützung geben oder Wartungsoperationen initiieren. Über den externen Browser 18 bestehen nur beschränkte Autorisationen. So kann über den externen Browser 18 nicht in den laufenden Betrieb der Dialysemaschine eingegriffen werden.

Der externe Browser 18 kann sich auch in einer Zentralstation des Hospitals befinden, in dem die ECB-Station 10 betrieben wird. Medzinisches Personal kann auf diese Weise die Behandlung überwachen, aktuelle oder frühere Behandlungen und Daten speichern oder auch ökonomische Leistungsdaten aufnehmen, wie beispielsweise Materialverbrauch, Wasser- oder Stromverbrauch.

Schließlich kann auch ein externer Berater über den Browser 18 die Behandlung verfolgen.

Bei dem Ausführungsbeispiel ist an das Datennetz LAN 3 über ein Netzwerk-Interface 19 ein externer Web-Server 20 angeschlossen. Der Web-Server 20 befindet sich in einer Workstation. In ihn können spezifische Patientenparameter-Sets und/oder Labordaten und/oder Inventurdaten von Medikamenten, Filtern, Einmal-Artikeln u.s.w. eingegeben werden. Der externe Web-Server 20 kann auch dazu dienen, an die ECB-Station 10 eine neue Sofware-Version zu übertragen oder den Inhalt der Datenbasis 12 zu erneuern oder zu aktualisieren. Schließlich kann der externe Web-Server 20 unter anderem dazu benutzt werden, patientenspezifische Parametersätze und/oder Behandlungsvorschriften von der ECB-Station 10 zu übernehmen.

Im Ausführungsbeispiel ist an das lokale Datennetz LAN 4 eine weitere ECB-Station 2-10 angeschlossen, die in gleicher Weise ausgebildet ist, wie die Station 10. Sie hat einen eigenen internen Browser 2-14 und internen Web-Server 2-13 sowie eine ECB-Einheit 2-11.

Von jedem Punkt des globalen Datennetzes sind prinzipiell die gleichen Daten, Informationen und Darstellungen erhältlich wie auf dem Bildschirm des Benutzer-Interface 15. Jedoch werden durch die unterschiedlichen Autorisierungen einige Befehle, die von außerhalb kommen, nicht ausgeführt und bestimmte Daten werden nicht angezeigt.

Bei dem vorliegenden Ausführungsbeispiel ist zusätzlich zu dem Benutzer-Interface 15 ein zweites Benutzer-Interface 15a vorgesehen, dem ein zweiter Browser 14a zugeordnet ist. Während das Benutzer-Interface 15 dem Klinikpersonal zur Verfügung steht, ist das Benutzer-Interface 15a für die Benutzung durch den Patienten vorgesehen. Dieser kann unter Ausnutzung der ECB-Station ohne wesentlichen zusätzlichen Aufwand die angeschlossenen Datennetze mitbenutzen, beispielsweise E-Mails verschicken, beliebige Computerarbeiten verrichten oder auf das Internet zugreifen.

## Patentansprüche

1. Extracorporales Blutbehandlungssystem mit einer ECB-Station (10), die eine ECB-Einrichtung (11), einen mit der ECB-Einrichtung (11) kommunizierenden internen Web-Server (13), einen mit dem internen Web-Server (13) kommunizierenden internen Browser (14) und ein mit dem internen Browser (14) kommunizierendes Benutzer-Interface (15) enthält, wobei der interne Browser (14) über ein Datennetz (WAN) mit externen Web-Servern (20) kommunizieren kann und/oder der interne Web-Server (13) über das Datennetz (WAN) mit externen Browsern (18) kommunizieren kann,
**dadurch gekennzeichnet,**
**dass** auch der Datenverkehr zwischen dem Benutzer-Interface (15) und der ECB-Einrichtung (11) über den internen Browser (14) abgewickelt wird.

2. Extracorporales Blutbehandlungssystem nach Anspruch 1, mit Mitteln, durch die eine entfernt von der ECB-Station (10) angeordnete Zentralstation über einen externen Browser (18) die Behandlungsparameter, Behandlungsergebnisse und/oder wirtschaftlich relevante Daten des ECB-Betriebes abruft und registriert.

3. Extracorporales Blutbehandlungssystem nach Anspruch 1 oder 2, mit Mitteln, durch die eine entfernt von der ECB-Station (10) angeordnete Service-Station über einen externen Browser (18) Behandlungsdaten bzw. Betriebsdaten aus der ECB-Einrichtung (11) abruft.

4. Extracorporales Blutbehandlungssystem nach einem der Ansprüche 1 bis 3, mit einem Web-Server (20) zur Übertragung maschinenbezogener, patientenbezogener oder medikamentenbezogener Daten aus einer entfernt von der ECB-Einrichtung (10) angeordneten Datenbasis an den internen Browser (14).

5. Extracorporales Blutbehandlungssystem nach Anspruch 4, mit Mitteln zur Einstellung bzw. Veränderung von Betriebsparametern der ECB-Einrichtung (11) anhand einiger der übertragenen Daten.

6. Extracorporales Blutbehandlungssystem nach einem der Ansprüche 1 bis 5, mit Mitteln, die veranlassen, dass der interne Web-Server (13) eine Autorisation für Parameter-Einstellungen, Informationszugriff und Steueroperationen in Abhängigkeit von der Identität des kommunizierenden Browsers (14,18,2-14) oder Web-Servers (13,20, 13-2) erteilt.

## Claims

1. An extracorporeal blood treatment system with an ECB station (10) comprising an ECB means (11), an internal web server (13) communicating with the ECB means (11), an internal browser (14) communicating with the internal web server (13), and a user interface (15) communicating with the internal browser (14), the internal browser (14) being adapted to communicate with external web servers (20) over a data net (WAN) and/or the internal web server (13) being adapted to communicate with external browsers (18) via the data net (WAN), **characterized in that** the data communication between the user interface (15) and the ECB means (11) is also effected via the internal browser (14).

2. The extracorporeal blood treatment system of claim 1, comprising means through which a central station remote from the ECB station (10) calls and registers treatment parameters, treatment results and/or economically relevant data of the ECB operation via the external browser (18).

3. The extracorporeal blood treatment system of claim 1 or 2, comprising means through which a service station remote from the ECB station (10) calls treatment data or operation data from the ECB station (11) via the external browser (18).

4. The extracorporeal blood treatment system of one of claims 1 to 3, comprising a web server for transmitting machine-related, patient-related or drug-related data are transmitted to the internal browser (14) from a data base remote from the ECB station (10).

5. The extracorporeal blood treatment system of claim 4, comprising means for setting or varying operation parameters of the ECB means (11) using some of the transmitted data.

6. The extracorporeal blood treatment system of one of claims 1 to 5, comprising means that cause the internal web server (13) to issue an authorization for parameter settings, information access and control operations depending on the identity of the communicating browser (14, 18, 2-14) or web server (13, 20, 13-2).

## Revendications

1. Système de traitement extracorporel du sang avec une station ECB (10) qui contient un dispositif ECB (11), un serveur Web interne (13) communiquant avec le dispositif ECB (11), un navigateur interne (14) communiquant avec le serveur Web interne (13) et une interface utilisateur (15) communiquant avec le navigateur interne (14), dans lequel le navigateur interne (14) peut communiquer par un réseau de données (WAN) avec des serveurs Web externes (20) et/ou le serveur Web interne (13) peut communiquer par le réseau de données (WAN) avec des navigateurs externes (18),
**caractérisé en ce que**
le trafic de données entre l'interface utilisateur (15) et le dispositif ECB (11) est également réalisé par l'intermédiaire du navigateur interne (14).

2. Système de traitement extracorporel du sang selon la revendication 1, avec des moyens par lesquels une station centrale éloignée de la station ECB (10) demande et enregistre, par l'intermédiaire d'un navigateur externe (18), les paramètres du traitement, les résultats du traitement et/ou les données économiquement intéressantes du fonctionnement ECB.

3. Système de traitement extracorporel du sang selon la revendication 1 ou 2, avec des moyens par lesquels une station de service éloignée de la station ECB (10) demande au dispositif ECB (11), par l'intermédiaire d'un navigateur externe (18), des données de traitement ou des données de fonctionnement.

4. Système de traitement extracorporel du sang selon l'une quelconque des revendications 1 à 3, avec un serveur Web (20) pour la transmission de données concernant une machine, un patient ou un médicament d'une base de données éloignée du dispositif ECB (10) au navigateur interne (14).

5. Système de traitement extracorporel du sang selon la revendication 4, avec des moyens pour régler ou modifier des paramètres de fonctionnement du dispositif ECB (11) à l'aide de quelques-unes des données transmises.

6. Système de traitement extracorporel du sang selon l'une quelconque des revendications 1 à 5, avec des moyens qui incitent le serveur Web interne (13) à délivrer une autorisation pour des réglages de paramètres, un accès aux informations et des opérations de commande en fonction de l'identité du navigateur (14, 18, 2-14) ou du serveur Web (13, 20, 13-2) communiquant.
